(19) Europäisches Patentamt
European Patent Office
Office européen des brevets

(11) EP 0 810 889 B1

(12) **EUROPÄISCHE PATENTSCHRIFT**

(45) Veröffentlichungstag und Bekanntmachung des Hinweises auf die Patenterteilung:
**27.06.2001   Patentblatt 2001/26**

(51) Int Cl.7: **A61L 27/00**

(21) Anmeldenummer: **96903901.5**

(86) Internationale Anmeldenummer:
**PCT/DE96/00322**

(22) Anmeldetag: **22.02.1996**

(87) Internationale Veröffentlichungsnummer:
**WO 96/25960 (29.08.1996 Gazette 1996/39)**

(54) **IMPLANTAT**

IMPLANT

IMPLANT

(84) Benannte Vertragsstaaten:
**AT BE CH DE DK FR GB IT LI NL SE**

(30) Priorität: **22.02.1995  DE 19506188**

(43) Veröffentlichungstag der Anmeldung:
**10.12.1997   Patentblatt 1997/50**

(73) Patentinhaber:
• **Lazarov, Miladin P.**
  **80797 München (DE)**
• **Mayer, Isabella**
  **80797 München (DE)**

(72) Erfinder:
• **Lazarov, Miladin P.**
  **80797 München (DE)**
• **Mayer, Isabella**
  **80797 München (DE)**

(74) Vertreter: **Schüssler, Andrea, Dr.**
**Kanzlei Huber & Schüssler**
**Truderinger Strasse 246**
**81825 München (DE)**

(56) Entgegenhaltungen:
**EP-A- 0 295 397          EP-A- 0 410 711**
**WO-A-95/17533**

• **STN International, File CAPLUS, Caplus accession no. 1992:639889, Kimura, Keizo et al: "Manufacture of prosthetic implants from titanium and its alloy", & JP,A2,04200557, 920721, Heisei**
• **STN International, File CAPLUS, Caplus accession no. 1989:219129, Kondo, Kazuo et al: "Ceramic- coated prosthetic implants",& JP,A2,63160666, 880704, Showa**

## Beschreibung

Die Erfindung betrifft ein Implantat der im Oberbegriff des Anspruchs 1 angegebenen Art.

Verschiedene künstliche Materialien werden als kurzfristiges oder längerfristiges Implantat für Diagnostik und Therapie in den menschlichen Körper eingebracht (Katheter, Sonden, Sensoren, Stents, künstliche Herzklappen, endotracheale Tuben u.a.). Die Materialwahl für diese Implantate ist abhängig von der Stabilität und Geometrie, die erforderlich ist, um eine bestimmte Funktion des Implantates zu gewährleisten. Um diesen Anforderungen in bezug auf die Funktion zu entsprechen, kann oft nicht genügend darauf Rücksicht genommen werden, ob diese Materialien bioverträglich sind. Daher ist es sinnvoll die Materialien, aus denen diese Implantate gefertigt werden, durch Beschichtungen zu veredeln, die mit Blut und Gewebe verträglich sind. Gefordert sind Beschichtungen, die das Gerinnungssystem nur in geringem Maße aktivieren, wenig körpereigene Abwehrreaktionen auslösen und damit die Ablagerung von Thromben und Biofilm an der Oberfläche des Implantates reduzieren. Eine Beschichtung ist für alle Materialien, die direkt in die Blutbahn eingebracht werden, sinnvoll, z.B. für Gefäßprothesen, Stents oder künstliche Herzklappen, aber auch für viele Implantate, die Kontakt mit Gewebe haben, z. B Herzschrittmacher oder Defibrillatoren oder alle anderen Implantate, die mit Körperflüssigkeiten in Berührung stehen, z.B. Gallengangsdrainagen, Katheter zur Ableitung von Harn bzw. Hirnflüssigkeit oder endotracheale Beatmungstuben. Die Blutverträglichkeit von Implantaten wird entscheidend von der Beschaffenheit ihrer Oberfläche beeinflußt. Für die Vermeidung von Thrombenbildung ("Antithrombogenität") ist zum Beispiel eine geringe Rauhtiefe notwendig, um die Anlagerung bzw. Zerstörung korpuskulärer Bestandteile des Blutes und die damit verbundene Aktivierung des Gerinnungssystemes zu verhindern. Desweiteren müssen jedoch auch direkte Ladungsaustauschprozesse zwischen gerinnungsspezifischen Proteinen und der Implantatoberfläche verhindert werden.

Es ist bekannt zur Erfüllung dieser Anforderungen Beschichtungen aus pyroliytischem Kohlenstoff als gebräuchlichem Werkstoff für Herzklappen einzusetzen. Daneben ist bekannt zur Verhinderung der Landungsaustauschprozesse zwischen den gerinnungsspezifischen Proteinen und der Implantatoberfläche halbleitende Materialien, zum Beispiel a-SiC:H als Implantatbeschichtung, zu verwenden (A. Bolz, M. Schaldach, "Haemocompatibility optimisation of implants by hybrid structuring", Med. & Biol. & Comput., 1993,31, S.123-130). Ferner ist bekannt $Ti_6Al_4V$ als Beschichtung einzusetzen (I. Dion, C. Baquey, J-R. Monties, P. Havlik, "Haemocompatibility of $Ti_6Al_4V$ alloy", Biomaterials, Vol. 4, 1993, S. 122-126). Auch auf dem Sektor der Polymerchemie wird eine Vielzahl von Kunststoffen untersucht, um nicht-haftende Oberflächen herzustellen. Auch in diesem Bereich konnte das Problem bisher nicht zufriedenstellend gelöst werden (R.F. Brady Jr, "Coming to an unsticky end", Nature, Vol 368, 1994, S. 16-17).

Ein Beschichtungsmaterial fur solartechnische Auwendungen ist in dem älteren Recht WO-A-95/17533 jezeigt.

Implantate mit einer Beschichtung aus Kohlenstoff, die eine poröse Struktur aufweisen, werden zwar den Ansprüchen an die Oberflächenbeschaffenheit gerecht, sie haben jedoch den Nachteil, daß ein Elektronentransfer durch Tunneln von besetzten, valenzbandartigen Zuständen des Proteins in freie Zustände des Festkörpers zu einer Spaltung des Fibrinogens im Blut führt. Die dabei entstehenden Fibrinmonomere polymerisieren und führen zu einem irreversiblen Thrombus. Implantate aus Rutilkeramik unterbinden zwar diese Ladungsaustauschprozesse, sie sind jedoch aufgrund der hohen Produktionskosten nicht zur Serienreife gelangt. Implantate mit einer Beschichtung aus amorphem Kohlenstoff (a-SiC:H) sind zwar kostengünstig herzustellen, aber der Nachteil des Materials besteht darin, daß es für bestimmte Anwendungen keine große Härte aufweist. Dieses Material wird derzeit mittels CVD-Verfahren hergestellt, die als zusätzliches Problem eine starke Erhitzung des Substrates erfordern, so daß eine Anwendung für eine große Anzahl von hitzeempfindlichen Grundmaterialien erschwert ist. Zusätzlich hat dieses Material eine feste Bandlücke und geringe Leitfähigkeit. Beide Eigenschaften führen eher zur Thrombenbildung anstatt sie zu verhindern.

Der Erfindung liegt deshalb die Aufgabe zugrunde, bei einem Implantat der eingangs genannten Gattung eine Oberfläche zu schaffen, mit der die Aktivierung der Blutgerinnung mit Thrombenbildung sowie die Bildung von Biofilm stark vermindert wird.

Diese Aufgabe wird durch ein Implanatat gemäß Patentanspruch 1 gelöst. Vorteilhafte Ausgestaltungen ergeben sich aus den Unteransprüchen. Weiter wird die Aufgabe durch die Merkmale des Patentanspruch 17 gelöst.

Als Implantat im Sinne der Erfindung soll jedes Gerät oder jede Vorrichtung verstanden werden, die in den tierischen oder menschlichen Körper implantiert oder länger- bzw. kurzfristig eingeführt oder an den tierischen oder menschlichen Körper angebracht werden kann. Als Beispiele sind zu nennen: Katheter, Sonden, Sensoren, Stents, künstliche Herzklappen, endotracheale Tuben, Herzschrittmacher.

Das Material für die Beschichtung des Implantats, also das Beschichtungsmaterial, enthält chemische Verbindungen zwischen einem oder mehreren Metallen der Gruppe IVA des Periodensystems, Stickstoff und Sauerstoff, wobei 2-45%, vorzugsweise 10-43%, ganz bevorzugt 20-35% des Volumens durch Leerräume (genannt voids), deren Größe im Bereich von $(0,4 \text{ nm})^3$ [$=0,064 \text{ nm}^3$] bis $(50 \text{ nm})^3$ [$=125000 \text{ nm}^3$], bevorzugt $(0,4 \text{ nm})^3$ bis $(20 \text{ nm})^3$, liegt, gebildet wird. Mit diesen Leeräumen sind nicht die in der Literatur (z.B. John A. Thornton, The microstructure of sputter-deposited coatings, J. Vac. Sci. Technol. A4 (6), 1986, S. 3059-3065) häufig beschriebenen Spalten gemeint, die durch kolumnares Schichtwachstum gebildet werden. Es handelt sich also nicht um die bekannten Spalten zwischen den Kolumnen, sondern vielmehr um Leerräume innerhalb dieser Säulen. Gerade diese führen zu den erfindungsspezifischen Eigenschaften. Das restliche Volumen des Beschichtungsmaterials (98-55%, vorzugsweise 90-57%, ganz bevorzugt 80-65%) weist eine Zusammensetzung des Metalls der Gruppe IVA des Periodensystems zu Stickstoff zu Sauerstoff wie 1:(0,1 bis 1,7):(0,1-1,7), vorzugsweise 1:(0,4 bis 1,2):(0,1 bis 1,2) auf. Das Material hat die Formel $MN_xO_y$, wobei "M" Metall der Gruppe IVA des Periodensytems bedeutet und x bzw. y die Werte 0,1 bis 1,7 haben. Die obigen Verhältnisse beziehen sich auf die Teilchenanzahl bzw. Molverhältnisse. Das Metall der Gruppe IVA des Periodensystems ist Titan, Zirkonium oder Hafnium oder eine Mischung von zweien oder den dreien Metallen, vorzugsweise Titan.

Bei den Größen der Leerräume ist es bevorzugt, daß diese im unteren Bereich, d.h. vorzugsweise nicht größer als $(15\,nm)^3$, auftreten. Das "restliche Volumen" des Materials umfaßt vorzugsweise eine oder mehrere der chemischen Verbindungen ausgewählt aus $MN_x$ (x = 0,7-1,2), $MO_x$ (x = 0,7-1,2), Magnelli-Phasen des M-O-Systems ($MnO_{2n-1}$), $MO_2$, $M_2N$ (mit M = Metall der Gruppe IVA des Periodensystems) sowie ca. 0-30%, vorzugsweise 0,5-5%, an Kohlenstoffverbindungen eines Metalls der Gruppe IVA des Periodensystems. Durch diese vorzugsweise zusätzlich enthaltenen Kohlenstoffverbindungen wird das Spektrum an Einsatzmöglichkeiten der Beschichtung erweitert und die Stabilität erhöht, was sich z.B. bei Urinkathetern gezeigt hat. Geringe Mengen an Titancarbiden als Verunreinigungen sind meist nicht störend, erlauben sogar eine billigere Herstellung der Beschichtung. Die Möglichkeit, daß im Beschichtungsmaterial die chemischen Phasen vorzugsweise in kristalliner oder in amorpher Form vorliegen können, verhindert die Anlagerung und Zerstörung korpuskulärer Bestandteile des Blutes, wodurch die damit verbundene Aktivierung des Gerinnungssystems verhindert wird. Das Beschichtungsmaterial wirkt deshalb einer Thrombusbildung entgegen, d.h. zeigt "antithrombogene" Eigenschaften.

Die Möglichkeit eine fraktale Größenverteilung der Leerräume einzuführen, erlaubt eine wesentliche Vergrößerung der Oberfläche wie sie vorzugsweise bei Schrittmacherelektroden verwendet wird. Eine größere Oberfläche erlaubt eine Verringerung der elektrischen Impedanz und damit eine längere Lebensdauer der Batterie eines Schrittmachers.

Das Beschichtungsmaterial weist weiter vorzugsweise auf, daß der Realteil der Brechzahl für die Röntgenwellenlänge von 0,0709 nm im Bereich von 0,9999984 bis 0,9999973 liegt. Die Massendichte des Beschichtungsmaterials liegt vorzugsweise im Bereich von 3,5 bis 5,4 $g/cm^3$, vorzugsweise 3,7 bis 4,5 $g/cm^3$, weiter bevorzugt von 3,8 bis 4,2 $g/cm^3$.

Als zusätzliche Metalle kann das Beschichtungsmaterial neben den Metallen der Gruppe IVA des Periodensystems auch Niob, Tantal, Wolfram, Molybdän oder deren Legierungen enthalten, was sich vorteilhaft auf die Korrosionsbeständigkeit der Beschichtung auswirkt.

Die Möglichkeit, daß das Material Wasserstoff enthält (gelöst oder bevorzugt gebunden), führt dazu, daß freie Bindungen in den amorphen Phasen abgesättigt werden. Damit wird eine für die Bioverträglichkeit günstigere Elektronenzustandsverteilung bewirkt.

Die Dicke der Beschichtung liegt vorzugsweise im Bereich von 3 nm bis 3 mm, weiter bevorzugt im Bereich von 10 nm bis 2 mm, ganz bevorzugt von 30 bis 71 nm.

Die Beschichtung hat bevorzugt einen spezifischen Widerstand im Bereich von 30 bis 30000 µΩ.cm, vorzugsweise 100 bis 6000 µΩ.cm, ganz bevorzugt 2000 bis 3000 µΩ.cm. Der spezifische Widerstand kann durch die Wahl des Leerraumanteils problemlos eingestellt werden. Ausgehend vom spezifischen Widerstand des Materials ohne Leerraum erhöht sich dieser beim Hinzufügen von Leerräumen. Zum Beispiel kann bei $TiN_{0,98}O_{0,2}$ der spezifische Widerstand bei 70 µΩ.cm liegen, wenn der Leerraumanteil bei 3% liegt und wächst bis auf Werte von 650 µΩ.cm, wenn der Leerraumanteil bei 40% liegt.

Die Beschichtung befindet sich als dünne Schicht auf einem als Implantat geeigneten Substrat. Dieses Substrat kann aus Kunststoff, z.B. Polyester, Polyamid, Polyurethan (PUR), Polyethylen (PE), Polytetrafluorethylen (PTFE) oder DACRON$^R$ sein oder aus einem Metall, wie Molybdän, Silber, Gold, Kupfer, Aluminium, Wolfram, Nickel, Chrom, Zirkonium, Titan, Hafnium, Tantal, Niob, Vanadium, Eisen oder deren Mischungen oder Legierungen sein. Die als dünne Schicht ausgebildete Beschichtung ist vorzugsweise auf eine rauhe Substratoberfläche aufgebracht, deren Rauhigkeit durch eine statistische Verteilung der Abweichungen vom mittleren Niveau gekennzeichnet ist und die Standardabweichung dieser Verteilung liegt im Bereich von 0 - 1500 nm, vorzugsweise 40-120 nm.

Die Beschichtung kann weiter mit mindestens einer weiteren dünnen Schicht, ausgewählt aus einem oder mehreren Oxiden, vorzugsweise $SiO_2$, $TiO_2$, $ZrO_2$, $HfO_2$, $Al_2O_3$, $Y_2O_2$, Niob-, Molybdän-, Wolfram- und Tantaloxiden beschichtet sein.

In einer bevorzugten Ausführungsform ist eine Zwischenschicht zwischen Substrat und Beschichtung vorgesehen, die eine höhere Haftfestigkeit bewirkt. Diese Zwischenschicht besteht aus einem Metall, vorzugsweise aus Chrom, Kupfer, Nickel, Molybdän, Tantal, Niob, Silber oder Legierungen dieser Metalle, oder einem Halbleiter.

Ein Einsprossen von körpereigenen Zellen, was insbesondere der Verankerung des Implantates, aber auch zur Anregung der Ausbildung einer physiologischen Oberfläche dient, kann durch die Zusammensetzung der Materialoberfläche gesteuert werden. Die Oberflächenbeschichtung kann auf viele verschiedene Grundmaterialien (Substrate), z.B. Metalle und Kunstoffe, mit unterschiedlicher Geometrie aufgebracht werden. Ein besonderer Vorteil der erfindungsgemäßen Implantate ist die kostengünstige Herstellung sowie daß die Beschichtung in Abhängigkeit von dem gewählten Verfahren auch auf Materialien erfolgen kann, die aufgrund ihrer besonderen Struktur keine Erwärmung tolerieren.

Die Beschichtung kann sowohl mittels CVD- und PVD-Verfahren, besonders bevorzugt jedoch mit PVD-Verfahren erfolgen. Insbesondere eignet sich zur Herstellung der erfindungsgemäßen Implantate das folgende Verfahren: Während des Abscheidens des Metalls der Gruppe IVA des Periodensystems auf einem als Implantat geeigneten Substrat entsteht durch Aufrechterhalten einer Gasatmosphäre, die mind. eine der Gassorten $N_2$, $O_2$, $CH_4$ und/oder Edelgase enthält, eine Oxid-, Nitrid- oder Carbidverbindung. Dabei wird die Kondensation der Metallteilchen auf einem beheizbaren oder kühlbaren Substrat über den Gesamtgasdruck $p_{tot}$, die Aufdampfrate r, die Substrattemperatur $T_{sub}$ und durch den Abstand $\ell$ der Metallquelle und dem Substrat so gesteuert, daß der Volumenanteil an Leerräumen 2 bis 45 Vol.-% beträgt, deren Größe im Bereich von $(0,4\ nm)^3$ bis $(50\ nm)^3$ liegt. Die Herstellungsparameter werden wie folgt gewählt:

- $T_{sub}$ = -5 bis 400°C,
- $\ell$ = 0,01 bis 1,5 m
- das Partialdruckverhältnis der eingeführten Gase $N_2$ und $O_2$: $(P_{N2}/P_{O2})$ = 1 bis 2000,
- $P_{tot}$ = 2 x $10^{-5}$ hPa-4 x $10^{-2}$ hPa und
- r = 0,01 bis 60 Nanometer/s.

Bei dem Herstellungsverfahren ist es notwendig, die Herstellungsparameter so einzustellen, daß der Anteil an Leerräumen vorhersehbar ist. Dies kann nach folgendem Vorgehen geschehen: für Substrattemperaturen im Bereich von vorzusgweise 100 bis 220 °C und einem Abstand der Verdampferquelle zu Substrat $\ell$ im Bereich von vorzugsweise 0,5 bis 1,2 m gilt:

Ein Volumenanteil von 34 % an Leerräumen wird erreicht, wenn

$$K = P_{tot} \cdot r/\ell = (1\ \text{bis}\ 3) \cdot 10^{-4}\ \frac{mBar\ nm}{sm}$$

und der Gesamtgasdruck $P_{tot}$ im Bereich von 0,7 x $10^{-3}$ hPa bis 2 x $10^{-2}$ hPa liegt.

Ein Volumenanteil von 20 % an Leerräumen wird erreicht, wenn im Bereich von

$$K = P_{tot} \cdot r/\ell = (0,2\ \text{bis}\ 0,5) \cdot 10^{-4}\ \frac{mBar\ nm}{sm}$$

gewählt wird. Volumenanteile zwischen 20 und 34 % können durch Wahl der Größe K nach folgender Gleichung eingestellt werden:

$$K = ((0,04\ \text{bis}\ 0,2) \cdot \text{gewünschter Anteil an Leerräumen} -0,7) \cdot 10^{-4}\ \frac{mBamm}{sm}$$

Damit hat man die Möglichkeit sowohl mit der Rate r, mit dem Gesamteindruck $P_{tot}$ und mit dem Abstand $\ell$ den gewünschten Leerraumanteil in dem erfindungsgemäßen Material zu erzielen.

Analog kann man für die Substrattemperaturen im Bereich von vorzugsweise 250 bis 400 °C und $\ell$ im Bereich von vorzugsweise 0,5 bis 1,2 m den Volumenanteil der Leerräume in der Schicht folgendermaßen steuern: Ein Volumenanteil von z.B. 40 % an Leerräumen wird erreicht wenn

$$K = (6\ \text{bis}\ 8) \cdot 10^{-4}\ \frac{mBar\ nm}{sm}$$

und $P_{tot}$ im Bereich von 2 x $10^{-2}$ hPa bis 4 x $10^{-2}$ hPa liegt. Wenn K im Bereich von

$$x\ K = (0,8\ \text{bis}\ 1,9) \cdot 10^{-4}\ \frac{mBar\ nm}{sm}$$

gewählt wird, ist der Volumenanteil der Leerräume 20 %. Um Werte zwischen 20 und 40 % Volumenanteile zu realisieren, muß K nach der Gleichung:

$$K = ((0{,}12 \text{ bis } 0{,}31) \cdot \text{gewünschter Anteil an Leerräumen} - 0{,}4) \cdot 10^{-4} \ \frac{\text{mBar nm}}{\text{sm}}$$

gewählt werden. Dazwischen liegende Volumenanteile können durch jeweils lineare Interpolation ermittelt werden. Kleine Volumenanteile an Leerräumen (2-20 %) werden bei kleinen Raten 0,01-0,1 nm/s und bei niedrigen Gasdrücken von $10^{-4}$ - $2 \times 10^{-4}$ mBar erreicht. Sehr große Leerraumanteile (> 40 %) werden bei hohen Totalgasdrücken > $4 \times 10^{-2}$ mBar erreicht: Bei diesen Gasdrücken kann das Material als lockerer Verbund vorliegen. Die Beschichtung erfolgt auf einem Substrat, das sich wie vorn definiert als Implant eignet.

Bei Kunststoffimplantaten muß $T_{sub}$ natürlich so gewählt werden, daß sich der Kunststoff nicht verändert, d. h. $T_{sub}$ sollte vorzugsweise 5-20 Kelvin unter der Umformungstemperatur des Kunststoffs liegen.

Die Abscheidung der Beschichtung auf dem Substrat erfolgt in einer üblichen Vakuumabscheidungsapparaturwie sie dem Fachmann auf diesem Fachgebiet geläufig ist.

Der Vorteil des Beschichtungsmaterials besteht im Vergleich zu den bisher verwendeten Materialien insbesondere darin, daß zwischen verschiedenen Zustandsformen (metallisch, dielektrisch) variiert werden kann. Damit kann das Material in seiner Zusammensetzung so eingestellt werden, daß es anziehende oderabstoßende Eigenschaften für Körperzellen, Bluteiweißbestandteile oder Mikroorganismen haben kann.

Bei dem Material gibt es einen Anteil an Leerräumen, dessen Anteil am Volumen beliebig gesteigert oder gesenkt werden kann. Durch den Leerraumanteil kann die Bandstruktur entsprechend den Anforderungen angepaßt werden, ohne die chemische Zusammensetzung zu verändern. So gehen die positiven Eigenschaften des Beschichtungsmaterials sehr schnell verloren, wenn die Leerraumgröße über $(50 \text{ nm})^3$ beträgt. Ferner kann durch die gezielte Wahl des Leerraumanteils das Einwachsen oder Anhaften von lebenden Zellen gesteigert oder vermindert werden. In diesem Zusammenhang ist es bevorzugt, daß die Beschichtung zusätzlich zu den schon beschriebenen Leerräumen im Bereich von $(0{,}4 \text{ nm})^3$ bis $(50 \text{ nm})^3$ auch größere Leerstellen über $(500 \text{ nm})^3$ enthält. Dies fördert das Einwachsen des Implantats in vorgegebene Zellstrukturen oder auch das Anwachsen von Zellen auf der beschichteten Oberfläche. Dies wird dadurch gefördert, daß das zu beschichtende Implantatmaterial (also das Substrat) eine rauhe Oberfläche besitzen darf. Die Oberflächenrauhigkeit ist vorzugsweise zwischen O-1500 µm.

Ein weiterer Vorteil besteht darin, daß dem Material Depotstoffe, vorzugsweise gerinnungshemmende Substanzen oder Antibiotika beigefügt werden können, die dann kontinuierlich aus dem Material freigesetzt werden.

Die Oberflächenbeschichtung bei dem erfindungsgemäßen Implantat ist deswegen besonders vorteilhaft, weil das beschriebene Material eine Leitfähigkeit besitzt, welche die Bildung von Raumladungen, die Fibrinogen aktivieren, verhindert. Zusätzlich weist das beschriebene Beschichtungsmaterial eine Elektronenzustandsdichte auf, die einen Elektronenstrom vom Fibrinogen zum Implantat nicht zuläßt. Auch diese Eigenschaft verhindert die Aktivierung des Fibrinogens. Diese positiven Eigenschaften des beschriebenen Materials werden auf zweifache Weise eingestellt. Einerseits durch Variation der Leerräume im beanspruchten Bereich und andererseits durch die Variation der chemischen Zusammensetzung. Mit beiden Möglichkeiten wird erreicht, daß sich die Elektronenzustandsdichte der Beschichtung auf dem energetischen Niveau der Proteinzustände im Blut befindet. Weiter erweist es sich als günstig, daß die Beschichtung derart ausgebildet ist, daß die Ladungsaustauschprozesse zwischen gerinnungsspezifischen Proteinen und der Implantatoberfläche verhindert werden.

Das Implantat kann zur Implantation oder kurz- bzw. langfristigen Einführung oder Anbringung in bzw. an den tierischen oder menschlichen Körper verwendet werden.

Die Erfindung wird nun weiter mit Bezug auf die Figuren beschrieben, von denen zeigen:

Fig.1: Schnitt durch die Beschichtung eines Implantats

Fig.2: UPS-Spektren von sechs Beschichtungen von Endotrachealtuben

Fig.3: Kontaktwinkelaufnahmen von 3 Modifikationen von $TiN_xO_y$ (mit x = 1,0 und y = 1,1, aber wechselnden Leerraumanteilen) zu Wasser. Bei (a) beträgt der Leerraumanteil 40%, bei (b) 22% und bei (c) 3%. Durch den Anteil der Leerräume werden die Hafteigenschaften gesteuert.

Die Erfindung wird nun weiter mit Bezug auf die Beispiele beschrieben:

**Beispiele:**

Beispiel 1:

Die Oberfläche einer Herzklappenprothese aus dem Grundwerkstoff Titan wurde mit einer Beschichtung aus $TiN_xO_y$ (x = 1,2 und y = 0,6; gemessen mittels Elastic Recoil Detection (ERD)) beschichtet. Die Herstellung der Beschichtung fand mittels reaktiver Verdampfung unter Auswahl folgender Parameter statt: Substrattemperatur = 250°C, Abstand zwischen Verdampfer und Substrat = 45 cm; Partialdruckverhältnis ($N_2$ zu $O_2$) = 1500; Totaldruck = 7 x $10^{-4}$ hPa; Aufdampfrate = 0,2 nm/s.

Der Leeraumanteil im Beschichtungsmaterial lag um 28% und die Leeraumgröße zwischen $(0,6 \text{ nm})^3$ und $(0,9 \text{ nm})^3$

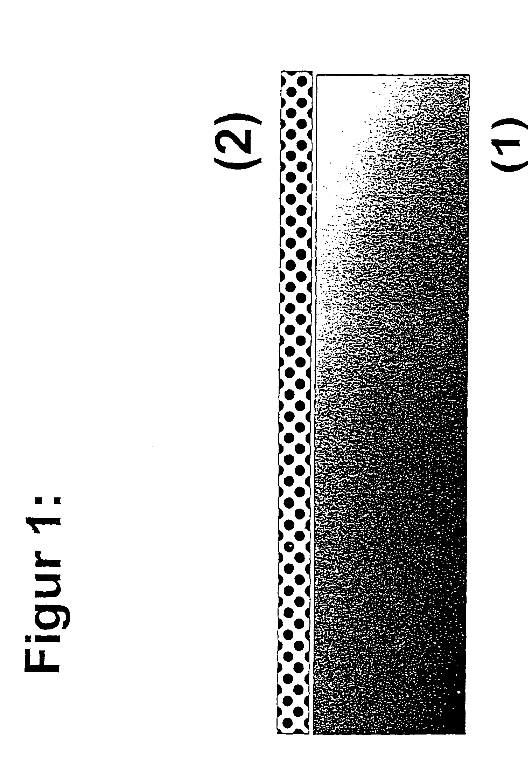

In Fig.1 ist ein Schnitt durch die beschichtete Herzklappenprothese gezeigt, wobei (1) die Oberfläche des Grundwerkstoffs und (2) die Beschichtung darstellt. Die Beschichtung (2) ist 3 µm dick.

Beispiel 2:

Es wurde die Beschichtung von sechs Endotrachealtuben vorgenommen. Die Herstellung der Beschichtung fand mittels reaktiver Verdampfung unter folgenden Parametern statt: Substrattemperatur = 250°C, Abstand zwischen Verdampfer (Metallquelle) und Substrat = 45 cm; Totaldruck = 7 x $10^{-4}$ hPa; Aufdampfrate = 0,2 nm/s. Variiert wurde das Partialdruckverhältnis ($N_2$ zu $O_2$) der zugeführten Gase. Der Leeraumanteil im Beschichtungsmaterial lag um 24% und die Leeraumgröße zwischen $(0,6 \text{ nm})^3$ und $(0,9 \text{ nm})^3$.

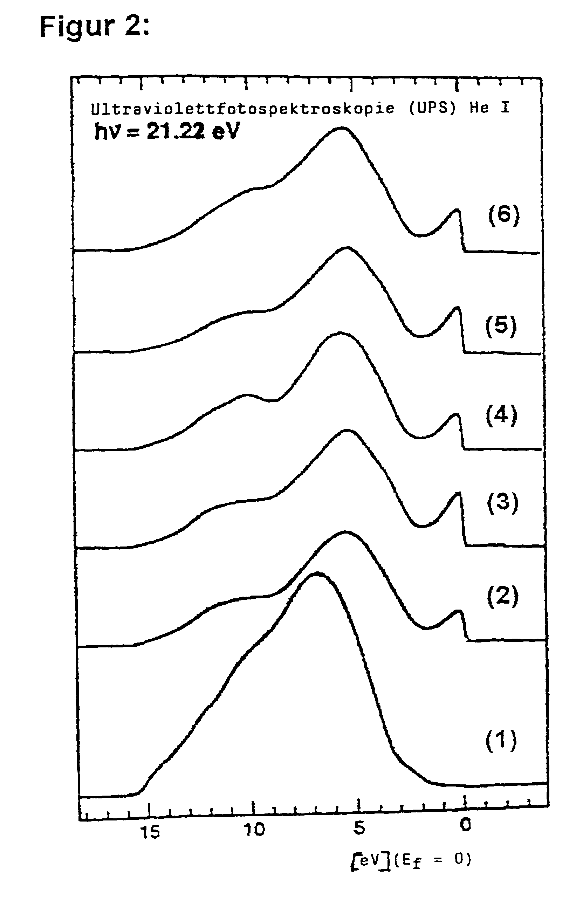

Von den sechs Beschichtungen wurden UPS-Spektren aufgenommen, die in Fig. 2 gezeigt sind:

Kurve (1): Beschichtung aus $TiN_xO_y$ mit x = 0,5 und y = 1,4, gemessen mittels Elastic Recoil Detection (ERD). Das Partialdruckverhältnis ($N_2$ zu $O_2$) betrug 120.

Kurve (2): Beschichtung aus $TiN_xO_y$ mit x = 0,9 und y = 1,2, gemessen mittels Elastic Recoil Detection (ERD). Das Partialdruckverhältnis ($N_2$ zu $O_2$) betrug 500.

Kurve (3): Beschichtung aus $TiN_xO_y$ mit x = 0,9 und y = 0,8, gemessen mittels Elastic Recoil Detection (ERD). Das Partialdruckverhältnis ($N_2$ zu $O_2$) betrug 900.

Kurve (4): Beschichtung aus $TiN_xO_y$ mit x = 0,9 und y = 0,2, gemessen mittels Elastic Recoil Detection (ERD). Das Partialdruckverhältnis ($N_2$ zu $O_2$) betrug 1200.

Kurve (5): Beschichtung aus $TiN_xO_y$ mit x = 1,4 und y = 0,3, gemessen mittels Elastic Recoil Detection (ERD). Das Partialdruckverhältnis ($N_2$ zu $O_2$) betrug 1600.

Kurve (6): Beschichtung aus $TiN_xO_y$ mit x = 1,5 und y = 0,15, gemessen mittels Elastic Recoil Detection (ERD). Das Partialdruckverhältnis ($N_2$ zu $O_2$) betrug 2000.

Bei den Beschichtungen, die den Kurven (2) bis (6) zugrundeliegen, erkennt man deutlich eine Fermikante, die mit der Leitfähigkeit korreliert ist.

Beispiel 3:

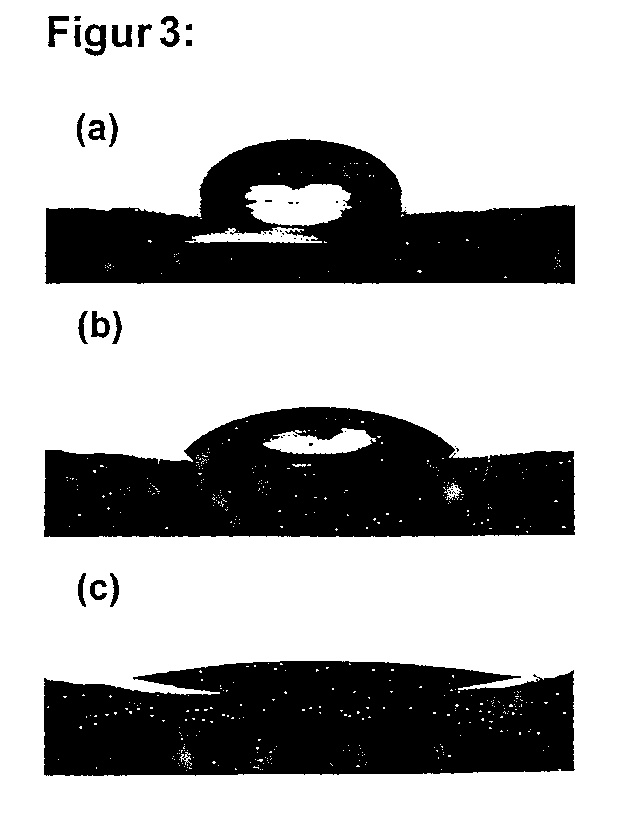

Es wurden drei verschiedene Beschichtungmaterialien aus $TiN_xO_y$ (x = 1,0, y = 1,1) mittels Vakuumabscheidung auf Endotrachealtuben aufgebracht und Kontaktwinkelaufnahmen der drei Beschichtungen vorgenommen. Die Ergebnisse sind in Fig. 3 gezeigt. (a) ist die Aufnahme einer Probe mit 40% Leeraumanteil, (b) einer Probe mit 22% Leeraumanteil und (c) einer Probe mit 3% Leeraumanteil.

Die zur Herstellung der Beschichtung gewählten Parameter waren:

- bei der Probe mit 40% Leeraumanteil:

Abstand zwischen Metallquelle und Substrat = 70 cm
Substrattemperatur $T_{sub}$ = 300 °C
Partialdruckverhältnis ($p_{N2}/p_{O2}$) = 1200
Aufdampfrate r = 0,01 nm/s
Gesamtgasdruck = 2 x $10^{-2}$ hPa
Es ergab sich eine Leeraumgröße zwischen $(0,8 \text{ nm})^3$ und $(2,8 \text{ nm})^3$.

- bei der Probe mit 22% Leeraumanteil:

Abstand zwischen Metallquelle und Substrat = 70 cm
Substrattemperatur $T_{sub}$ = 300 °C

Partialdruckverhältnis ($p_{N2}/p_{O2}$) = 1200
Aufdampfrate r = 0,25 nm/s
Gesamtgasdruck = 8 x 10$^{-4}$ hPa

Es ergab sich eine Leerraumgröße zwischen (0,6 nm)$^3$ und (0,9 nm)$^3$.

- bei der Probe mit 3% Leerraumanteil:

Abstand zwischen Metallquelle und Substrat = 70 cm
Substrattemperatur $T_{sub}$ = 300 °C
Partialdruckverhältnis ($p_{N2}/p_{O2}$) = 1200
Aufdampfrate r = 0,7 nm/s
Gesamtgasdruck = 2 x 10$^{-4}$ hPa

Es ergab sich eine Leerraumgröße zwischen (0,4 nm)$^3$ und (0,8 nm)$^3$.

Wie aus Fig. 3 ersichtlich ist, können durch die Variation des Leerraumanteils die Hafteigenschaften der Beschichtung gesteuert werden.

## Patentansprüche

1. Implantat, **dadurch gekennzeichnet,** daß ein ImplantatSubstrat aus Metall, Kunststoff oder deren Mischungen oder Legierungen mit einer dünnen Schicht eines Beschichtungsmaterial beschichtet ist, welches chemische Verbindungen zwischen einem oder mehreren Metallen (M) der Gruppe IV A des Periodensystems [=Ti, Zr, Hf], Stickstoff (N) und Sauerstoff (O) enthält, wobei in dem Beschichtungsmaterial 2 bis 45% des Volumens durch Leerräume (genannt voids) gebildet werden, deren Größe im Bereich von (0.4 nm)$^3$ bis (50 nm)$^3$ liegt, und das restliche Volumen eine Zusammensetzung eines Metalls der Gruppe IV A des Periodensystems zu Stickstoff zu Sauerstoff von 1:(0.1 bis 1.7): (0.1 bis 1.7) aufweist, wobei ein Material mit der Formel $MN_xO_y$ (mit x,y = 0.1-1.7) entsteht.

2. Implantat nach Patentanspruch 1, dadurch gekennzeichnet, daß das Beschichiungsmaterial zusätzlich eine oder mehrere der folgenden chemischen Verbindungen umfaßt:

- $MN_x$ mit x = 0.7 bis 1.2
- $MO_x$ mit x = 0.7 bis 1.2
- Magnelli-Phasen des M-O-Systems ($M_nO_{2n-1}$),
- $MO_2$
- $M_2N$

wobei M ein Metall der Gruppe IV A des Periodensystems ist.

3. Implantat nach einem oder mehreren der Patentansprüche 1 bis 2,
dadurch gekennzeichnet, daß in dem Beschichtungsmaterial kleine Mengen an Kohlenstoffverbindungen eines Metalls der Gruppe IV A des Periodensystems enthalten sind.

4. Implantat nach einem oder mehreren der Patentansprüche 1 bis 3,
dadurch gekennzeichnet, daß die Verbindungen in dem Beschichtungsmaterial in kristalliner oder in amorpher Form auftreten können.

5. Implantat nach einem oder mehreren der Patentansprüche 1 bis 4,
dadurch gekennzeichnet, daß der Realteil der Brechzahl des Beschichtungsmaterials für die Röntgenwellenlänge von 0.0709 nm im Bereich von 0.9999984 bis 0.9999973 liegt.

6. Implantat nach einem oder mehreren der Patentansprüche 1 bis 5,
dadurch gekennzeichnet, daß die Massendichte der Beschichtung im Bereich von 3,5 bis 5,4 g/cm$^3$ liegt.

7. Implantat nach einem oder mehreren der Patentansprüche 1 bis 6,
dadurch gekennzeichnet, daß neben den Metallen der Gruppe IV A des Periodensystems auch Niob, Tantal, Wolfram, Molybdän oder eine Legierung dieser Metalle enthalten ist.

**8.** Implantat nach einem oder mehreren der Patentansprüche 1 bis 7,
dadurch gekennzeichnet, daß das Beschichtungsmaterial Wasserstoff enthält.

**9.** Implantat nach einem oder mehreren der Patentansprüche 1 bis 8,
dadurch gekennzeichnet, daß die Schichtdicke des Beschichtungsmaterials auf dem Substrat im Bereich von 3 nm bis 3 mm liegt.

**10.** Implantat nach einem oder mehreren der Patentansprüche 1 bis 9,
dadurch gekennzeichnet, daß der spezifische Widerstand im Bereich von 30 bis 300 000 $\mu\Omega$cm liegt.

**11.** Implantat nach einem oder mehreren der Patentansprüche 1 bis 10,
dadurch gekennzeichnet, daß das Beschichtungsmaterial als dünne Schicht auf einer rauhen Substratoberfläche aufgebracht ist, deren Rauhigkeit durch eine statistische Verteilung der Abweichungen von einem mittleren Niveau gekennzeichnet ist und die Standardabweichung dieser Verteilung im Bereich von 0 bis 1500 $\mu$m liegt.

**12.** Implantat nach einem oder mehreren der Patentansprüche 1 bis 11,
dadurch gekennzeichnet, daß die dünne Beschichtungsschicht mit mindestens einer weiteren dünnen Schicht aus einem oder mehreren Oxiden ausgewählt aus $SiO_2$, $TiO_2$, $ZrO_2$, $HfO_2$, $A\ell_2O_3$ oder $Y_2O_3$, sowie Niob-, Molybdän-, Wolfram- oder Tantaloxiden beschichtet ist.

**13.** Implantat nach einem oder mehreren der Patentansprüche 1 bis 12,
dadurch gekennzeichnet, daß zwischen Substrat und Beschichtung mindestens eine weitere dünne Schicht vorzugsweise aus einem Metall oder Halbleiter eingeführt ist.

**14.** Implantat nach einem oder mehreren der Patentansprüche 1 bis 13,
dadurch gekennzeichnet, daß das Beschichtungsmaterial auf einem metallischen Substrat aus Molybdän, Silber, Gold, Kupfer, Aluminium, Wolfram, Nickel, Chrom, Zirkonium, Titan, Hafnium, Tantal, Niob, Vanadium, Eisen und deren Mischungen oder Legierungen aufgebracht ist.

**15.** Implantat nach einem oder mehreren der Patentansprüche 1 bis 13,
dadurch gekennzeichnet, daß das Beschichtungsmaterial auf einem Kunststoff-Substrat aus Polyester, Polyamid, Polyurethan (PUR), Polyethylen (PE), Polytetrafluorethylen (PTFE) oder DACRON$^R$ aufgebracht ist.

**16.** Implantat nach einem oder mehreren der Patentansprüche 1 bis 15,
dadurch gekennzeichnet, daß das Beschichtungsmaterial zusätzlich zu den Leerräumen im Bereich von $(0,4\ nm)^3$ bis $(50\ nm)^3$ auch Leerstellen größer als $(500\ nm)3$ aufweist.

## Claims

**1.** An implant, characterized in that an implant substrate made of metal, plastics or the mixtures or alloys thereof is coated with a thin layer of a coating material containing chemical compounds between one or more metals (M) of group IV A of the periodic system [= Ti, Zr, Hf], nitrogen (N) and oxygen (O), 2 to 45 % of the volume being formed in the coating material by voids whose size ranges from $(0.4\ nm)^3$ to $(50\ nm)^3$, and the residual volume having a composition of a metal of group IV A of the periodic system to nitrogen to oxygen of 1:(0.1 to 1.7):(0.1 to 1.7), a material with formula $MN_xO_y$ (wherein x,y = 0.1-1.7) forming.

**2.** The implant according to claim 1, characterized in that the coating material additionally comprises one or more of the following chemical compounds:

- $MN_x$, wherein x = 0.7 to 1.2
- $MO_x$, wherein x = 0.7 to 1.2
- magnelli phases of the M-O system ($M_nO_{2n-1}$),
- $MO_2$
- $M_2N$

wherein M is a metal of group IV A of the periodic system.

3. The implant according to one or more of claims 1 to 2, characterized in that the coating material contains small amounts of carbon compounds of a metal of group IV A of the periodic system.

4. The implant according to one or more of claims 1 to 3, characterized in that the compounds may occur in crystalline or amorphous form in the coating material.

5. The implant according to one or more of claims 1 to 4, characterized in that the real part of the refractive index of the coating material ranges from 0.9999984 to 0.9999973 for the X-ray wavelength of 0.0709 nm.

6. The implant according to one or more of claims 1 to 5, characterized in that the mass density of the coating ranges from 3.5 to 5.4 $g/cm^3$.

7. The implant according to one or more of claims 1 to 6, characterized in that niobium, tantalum, tungsten, molybdenum or an alloy of these metals is contained in addition to the metals of group IV A of the periodic system.

8. The implant according to one or more of claims 1 to 7, characterized in that the coating material contains hydrogen.

9. The implant according to one or more of claims 1 to 8, characterized in that the layer thickness of the coating material on the substrate ranges from 3 nm to 3 mm.

10. The implant according to one or more of claims 1 to 9, characterized in that the specific resistance ranges from 30 to 300,000 $\mu\Omega cm$.

11. The implant according to one or more of claims 1 to 10, characterized in that the coating material is applied as a thin layer on a rough substrate surface whose roughness is characterized by a random distribution of the deviations from a mean level and the standard deviation of this distribution ranges from 0 to 1500 $\mu m$.

12. The implant according to one or more of claims 1 to 11, characterized in that the thin coating layer is coated with at least one further thin layer comprising one or more oxides selected from the group consisting of $SiO_2$, $TiO_2$, $ZrO_2$, $HfO_2$, $Al_2O_3$ or $Y_2O_3$ and niobium, molybdenum, tungsten or tantalum oxides.

13. The implant according to one or more of claims 1 to 12, characterized in that at least one further thin layer, preferably consisting of a metal or semiconductor, is inserted between substrate and coating.

14. The implant according to one or more of claims 1 to 13, characterized in that the coating material is applied onto a metallic substrate consisting of molybdenum, silver, gold, copper, aluminum, tungsten, nickel, chromium, zirconium, titanium, hafnium, tantalum, niobium, vanadium, iron and the mixtures or alloys thereof.

15. The implant according to one or more of claims 1 to 13, characterized in that the coating material is applied onto a plastics substrate consisting of polyester, polyamide, polyurethane (PUR), polyethylene (PE), polytetrafluoroethylene (PTFE) or DACRON[R].

16. The implant according to one or more of claims 1 to 15, characterized in that the coating material also comprises voids greater than $(500 nm)^3$ in addition to the voids ranging from $(0.4 nm)^3$ to $(50 nm)^3$.

## Revendications

1. Implant caractérisé en ce qu'un substrat d'implant en métal, en matière plastique ou à base de mélanges ou d'alliages de ceux-ci, est revêtu d'une couche mince d'un matériau de revêtement qui contient des composés chimiques formés d'un ou plusieurs métaux (M) du groupe IV A du tableau périodique [= Ti, Zr, Hf], d'azote (N) et d'oxygène (O), de 2 à 45 % du volume du matériau de revêtement étant formés par des vides (appelés "*voids*") dont la taille est comprise entre $(0,4 nm)^3$ et $(50 nm)^3$, et le volume restant présentant un rapport d'un métal du groupe IV A du tableau périodique à l'azote à l'oxygène de 1 : (0,1 à 1,7) : (0,1 à 1,7), et on forme un matériau ayant la formule $MN_xO_y$ (avec x, y = 0,1 à 1,7).

2. Implant selon la revendication 1, caractérisé en ce que le matériau de revêtement comprend, en outre, un ou plusieurs des composés chimiques suivants :

- $MN_x$ avec x = 0,7 à 1,2,
- $MO_x$ avec x = 0,7 à 1,2,
- phases de Magnelli du système M-O ($M_nO_{2n-1}$),
- $MO_2$,
- $M_2N$,

M étant un métal du groupe IV A du tableau périodique.

3. Implant selon une ou plusieurs des revendications 1 et 2, caractérisé en ce que le matériau de revêtement contient des petites quantités de composés de carbures d'un métal du groupe IV A du tableau périodique.

4. Implant selon une ou plusieurs des revendications 1 à 3, caractérisé en ce que les composés dans le matériau de revêtement peuvent apparaître sous une forme cristalline ou amorphe.

5. Implant selon une ou plusieurs des revendications 1 à 4, caractérisé en ce que la partie réelle de l'indice de réfraction du matériau de revêtement pour la longueur d'onde des rayons X de 0,0709 nm est comprise entre 0,9999984 et 0,9999973.

6. Implant selon une ou plusieurs des revendications 1 à 5, caractérisé en ce que la masse volumique du revêtement est comprise entre 3,5 et 5,4 $g/cm^3$.

7. Implant selon une ou plusieurs des revendications 1 à 6, caractérisé en ce que, en plus des métaux du groupe IV A du tableau périodique, il contient aussi du niobium, du tantale, du tungstène, du molybdène ou un alliage de ces métaux.

8. Implant selon une ou plusieurs des revendications 1 à 7, caractérisé en ce que le matériau de revêtement contient de l'hydrogène.

9. Implant selon une ou plusieurs des revendications 1 à 8, caractérisé en ce que l'épaisseur de la couche du matériau de revêtement sur le substrat est comprise entre 3 nm et 3 mm.

10. Implant selon une ou plusieurs des revendications 1 à 9, caractérisé en ce que la résistance spécifique est comprise entre 30 et 300 000 $\mu\Omega$cm.

11. Implant selon une ou plusieurs des revendications 1 à 10, caractérisé en ce que le matériau de revêtement est appliqué sous la forme d'une couche mince sur une surface rugueuse du substrat dont la rugosité est caractérisée par une distribution statistique des écarts par rapport à un niveau moyen, et l'écart type de cette distribution est compris entre 0 et 1500 $\mu$m.

12. Implant selon une ou plusieurs des revendications 1 à 11, caractérisé en ce que la couche mince de revêtement est revêtue d'au moins une autre couche mince formée d'un ou plusieurs oxydes choisis parmi $SiO_2$, $TiO_2$, $ZrO_2$, $HfO_2$, $Al_2O_3$ ou $Y_2O_3$, ainsi que des oxydes de niobium, de molybdène, de tungstène ou de tantale.

13. Implant selon une ou plusieurs des revendications 1 à 12, caractérisé en ce qu'on introduit au moins une couche mince supplémentaire, de préférence formée d'un métal ou d'un semiconducteur, entre le substrat et le revêtement.

14. Implant selon une ou plusieurs des revendications 1 à 13, caractérisé en ce qu'on applique le matériau de revêtement sur un substrat métallique à base de molybdène, argent, or, cuivre, aluminium, tungstène, nickel, chrome, zirconium, titane, hafnium, tantale, niobium, vanadium, fer et des mélanges ou alliages de ceux-ci.

15. Implant selon une ou plusieurs des revendications 1 à 13, caractérisé en ce qu'on applique le matériau de revêtement sur un substrat en matière plastique formée de polyester, polyamide, polyuréthanne (PUR), polyéthylène (PE), polytétrafluoroéthylène (PTFE) ou DACRON[R].

16. Implant selon une ou plusieurs des revendications 1 à 15, caractérisé en ce que le matériau de revêtement présente, en plus des vides dans la plage de $(0,4 \text{ nm})^3$ à $(50 \text{ nm})^3$, aussi des vides supérieurs à $(500 \text{ nm})^3$.

# Figur 1:

(2)

(1)

# Figur 2:

Ultraviolettfotospektroskopie (UPS) He I
hv = 21.22 eV

(6)

(5)

(4)

(3)

(2)

(1)

15    10    5    0

[eV] ($E_f$ = 0)

# Figur 3:

(a)

(b)

(c)